# EUROPEAN PATENT SPECIFICATION

(11) **EP 2 186 509 B1**
(45) Date of publication and mention of the grant of the patent: **01.12.2010**
(21) Application number: 08750919.6
(22) Date of filing: 13.05.2008
(51) Int. Cl.: A61K 9/08, A61K 31/05, A61K 47/14, A61K 47/10, A61K 9/107

(54) **PROPOFOL TRANSPARENT ANAESTHETIC SOLUTION WITH LOW VENOUS IRRITATION**
TRANSPARENTE PROPOFOL-NARKOSELÖSUNG MIT GERINGER VENENREIZUNG
SOLUTION ANESTHÉSIANTE TRANSPARENTE À BASE DE PROPOFOL AVEC UN SEUIL D'IRRITATION INTRAVEINEUSE FAIBLE.

(30) Priority: 14.05.2007 AR P070102080
(43) Date of publication of application: 19.05.2010
(73) Proprietor: Quiroga, Ciriaco, 1550-240 Lisbon (PT)
(72) Inventor: Quiroga, Ciriaco, 1550-240 Lisbon (PT)
(74) Representative: Fiussello, Francesco
(86) International application number: PCT/IB2008/001178
(87) International publication number: WO 2008/139313

(56) References cited:
- WO-A-00/78301
- WO-A-2005/079758
- WO-A-2007/006334
- US-A1- 2004 067 919
- US-A1- 2005 004 234

## Description

### Background:

For more than fifty years, sodium thiopental has been used as intravenous anesthetic induction agent.

From 1935 until approximately 1990, a variety of intravenous induction medications has been introduced, which without displacing tiobarbiturate from its leading and predominant position, showing its relative advantages and disadvantages, illustrated in the following Table:

| **Effects** | **TIOP** | **MTOX** | **PRPN** | **ALTS** | **KET** | **ETOM** | **PRPFL** |
|---|---|---|---|---|---|---|---|
| Latency | + + | + + | + | + | + | + | + + |
| Awakening | + | + | | + | | | + |
| Excitation | - | + | + | + | +++ | ++ | - |
| Analgesia | - | - | | | | - | |
| Nausea & vomiting | - | - | ++ | + | + | + | - |
| Delirium | - | - | - | - | +++ | + | - |
| Injection-related pain | + | + | - | - | - | + | +++ |

| | | | | | | | |
|---|---|---|---|---|---|---|---|
| TIOP = Thiopental MTOX = Methohexital PRPN = Propanidine ALTS = Althesin KET = Cetamide ETOM = Etomidate PRPFL = Propofol + = Mild incidence ++ = Regular incidence +++ = Strong incidence | | | | | | | |

The most important qualities derived from the chemical composition of each inductor, as well as its distribution and redistribution, appears on the above mentioned Table, together with the most significant effects on the respiratory, cardiovascular and neurological systems. The analgesic or anti-analgesic effects of each inductor, as well as its drug interactions with other medication (muscle relaxants) were also compared.

As regards to these factors, the injection-related pain threshold is a very important factor to be considered, since it is an extremely undesirable effect, that patients perceives and remembers well before losing consciousness.

In 1989, the FDA (Food and Drug Administration) approved PROPOFOL as inductor, which rapidly gained popularity due to its safety to the above mentioned systems, and to its fast metabolism and elimination as well. However, there is still a serious disadvantage: as it appears on the above mentioned Table, it presents a very low threshold of pain: it has been verified that after being injected with Propofol, 80% of the patients had a rejection to the acute pain.

Propofol (2,6-diisopropylphenol) is a phenolic compound, of little solubility in water, that is presented as an emulsion made of soybean oil, glycerol and egg lecithin. Being this compound usually sold as sterile ampoules, without preservatives, with the above mentioned inactive ingredients, it allows the bacterial growth and the production of endotoxins.

These characteristics, as regards Propofol itself and the emulsions for its administration, imply that its intravenous administration is painful and the shelf-life of the product, once the ampoule is opened, is very short (approximately 8 hours).

Efforts have been made to eliminate or palliate such defect, but no significant result has been achieved up to now. Among the methods adopted to reduce the pain caused by Propofol, the following methods may be mentioned:
□ Warming of known emulsions;
□ Cooling of the emulsions;
□ Mix the emulsions with human blood;
□ Addition of lidocaine;
□ Injection through the veins of the forearm;
□ Injection of lidocaine, procaine, prilocaine;
□ Prior administration with methachlorpropamide, Fentanyl, ketorolac;
□ Add glucose or intralipid to the emulsion;
□ Administer the inductor mixed with fast venoclysis.

None of these efforts, however, have been effective, only some of these methods have partially prevented pain and the rest of them have completely failed. In fact, injection-related pain of Propofol is apparently caused by the formulation of this inductor, and it would be highly desirable to change said formulation to convert it into a substantially painless formulation, without modifying its hypnotic effect.

Indeed, the emulsions currently known of Propofol, as it was above mentioned, have the following problems:
✔ *They are very irritating;*
   The reason is that Propofol is an oil-based substance and therefore, it builds emulsions with known vehicles, making it a formulation with a very low solubility; the fluids of this solution are dense and not homogeneous, in addition, Propofol itself is irritating.
✔ *The known emulsions of Propofol do not have an acceptable level of transparency;*
   The transparency of the final product is particularly important, since it allows to see that there are not any lumps, suspensions or coagula on it, which may even cause thrombi in patients. Known Propofol emulsions are opaque and white-milky.
✔ *They are bacterial culture broth;*
   Known compositions contain soybean oil, glycerol, egg lecithin. This combination of substances is a base of proliferation and growth of bacteria and microorganisms, which imply that, after the package has been opened, the known emulsions of Propofol have a very short shelf-life or else a significant instability.

### Subject matters of this invention:

The subject matter of this invention is an injectable anesthetic solution of Propofol, water soluble, and substantially painless.

A characteristic of the subject matter of this invention is that this injectable water soluble solution is completely transparent, without lumps, coats or sediments in suspension.

Another characteristic of the subject matter of this invention is that this injectable solution of Propofol reduces the capacity of its components to any proliferation and growth of microorganisms and bacteria.

Finally, a further characteristic of the subject matter of this invention is that said injectable anesthetic solution of Propofol may allow a monitoring function throughout its manufacturing and packaging process to detect the presence of foreign bodies (foreign particles, traces of glass, etc.).

### Brief summary of the invention:

TRANSPARENT ANESTHETIC SOLUTION OF PROPOFOL WITH LOW INCIDENCE OF VENOUS IRRITATION, composed by at least 90% to 55% of Propofol, dissolved in 10% to 45% Macrogol 15 hydroxyestearate (Solutol).

### Detailed description of this invention:

In order to determine this invention, the following examples and their explanations are attached hereto together with their descriptions; the examples are presented as one of the many possibilities of manufacture of the invention, therefore no restriction should be assigned to it. The possible media, equivalent to what has been explained above must be included within the scope of protection for this invention. The extent of this invention shall be determined by the first claim attached to the chapter corresponding to Claims.

Solutol HS 15 (commercially registered by BASF AG, Germany) is a Macrogol 15 hydroxyestearate, i.e. mono and di-ester of polyglycol 12-hydroxyestearate with 30% of polyethylene glycol. Kept at room temperature, it is an off-white yellowish paste, which turns liquid at 30° C. It is neutral and some viscous in water.

Solutol HS 15^{®} is not usually soluble in oil-based liquids such as liquid paraffin.

It may be used in watery parenteral solutions with Vitamin A, D, E and K and other quantities of active pharmaceutical lipophilic agents.

However, its use as a vehicle which makes oil-based substances soluble, such as Propofol, is not yet known.

It has been surprisingly found that it is possible to obtain a solution of solubilized Propofol at room temperature with 10% to 45% of Solutol HS 15^{®}, preferably with 15% to 35% of Solutol HS 15^{®}, which is absolutely transparent.

It has been even more surprising to find that this solution provides an intravenous anesthetic injection, that keeps its anesthetic properties and reduces its irritability factor, with minor allergic reactions, obtaining thus a solution with a high shelf-life, without creating culture broth for microorganisms, bacteria and endotoxins.

### Examples of methods for obtaining the solution, subject matter of this invention:

Several laboratory assays have been made with two groups of crossbreed dogs, with an average weight of 25 kg.

The population of animals was sub-divided into two batches:
a) A first batch named ASA I with 15 animals,
b) A second batch named ASA II with 15 animals.

The animals were randomly chosen and the totality of the population was pre-medicated with acepromacine (0.05 mg/kg i.v.) and morphine (0.25 mg/kg) i.v., 5 minutes before the anesthetic induction.

Intravenous Propofol was administered in increasing doses until achieving unconsciousness and an anesthetic status compatible with the oro-tracheal tubing, after which the animals were connected to an anesthetic circuit and kept with isofluorane.

The ASA I group was administered Propofol 1% in a traditional emulsion:

| | | | |
|---|---|---|---|
| - | Propofol | 10.815 | mg/ml |
| - | EDTA | 0.0515 | mg/ml |
| - | Soybean oil | 100 | mg/ml |
| - | Glycerol | 25 | mg/ml |
| - | Egg lecithin | 8 | mg/ml |
| - | Alpha-tocopherol | 0,1 | mg/ml |
| - | Sodium Oleate | 0 a 0.30 | mg/ml |

And the ASA II group was induced with the following solution, subject matter of the invention:

| | | | |
|---|---|---|---|
| - | Propofol | 10.815 | mg/ml |
| - | EDTA | 0.0515 | mg/ml |
| - | Solutol HS 15^{®} | 2.7037 | mg/ml |

Solutol HS 15^{®} used is equivalent to 25% Propofol.

For all these cases the dose necessary was registered to promote the induction and the time required to carry out the oro-tracheal tubing. Animal behavior was evaluated during the administration of the different formulations of Propofol to observe signs of pain, irritation or discomfort.

The retraction of the limb used for the intravenous administration of the inductor or the vocalization were the pre-established signs to consider the formulation painful or irritating.

The following parameters were registered: heart rate (HR), breathing rate (BR), systolic blood pressure, diastolic and media blood pressure (SBP, DBP, MBP), end tidal CO₂ concentration (ETCO₂) and pulse oximetry (SpO₂).

The data were collected before (T0) and 5 minutes after (T5) the administration of the inductor. The totality of the components of both groups was evaluated up to 48 hours after the procedure to observe late adverse effects or signs of irritation of the vein that was used for the administration of the anesthetic solution.

The results are expressed as the media ± STG. The data were analyzed by means of a non parametric test for matched pair samples (Wilcoxon matched pair test). The differences are considered significant if p<0,05.

The following table of results has allowed to show that ASA II group, to which the solution of the invention was administered, has not showed venous irritation; the dog behavior was mild and no problems where observed, with a regular heart rate.

In all these cases, the solution of the invention was analyzed and the contents of the ampoules were observed against the light to verify the absence of lumps or foreign bodies, since said ampoules were transparent.

After these analysis and observations, It could be verified a longer shelf-life of the product and a higher stability, in view of a remarkable decrease in the speed of the reproduction of bacteria or microorganisms in the solution.

### Table of Results:

| **Population** | **Propofol 1%** | **Solution subject matter of the invention 1%** |
|---|---|---|
| Number of the sample (n) | 10 | 10 |
| Age (years) | 4.6 ± 2.2 (range 0.8 - 8) | 3.3 ± 2.4 (range 0.7-9) |
| Weight (Kg) | 18,2 ± 6.6 (range 11- 19) | 16.0 ± 3.5 (range 12-19.6) |
| Ratio M:H | 4:6 | 3:7 |

| **Monitoring** | **Propofol 1%** | **Solution subject matter of the invention 1%** |
|---|---|---|
| Dose (mg/Kg) | 4.5 ± 0.09 | 4.3 ± 0.9 |
| Time of injection (sec) | 16.2 ± 4.2 | 15.7 ± 4.8 |
| Latency for E tubing (sec) | 47.3 ± 11.6 | 48.7 ± 12.7 |

| **Parameter** | **Propofol 1%** | | **Solution subject matter of the invention 1%** | |
|---|---|---|---|---|
| | **T0** | **T5** | **T0** | **T%** |
| HR (lat/min) | 95 ± 16.2 | 91.7 ± 5.9 | 103.0 ± 30.7 | 87.2 ± 25.5 |
| SBP (mm Hg) | 139.0 ±26.0 | 122.3 ± 17 | 130.2 ± 10.8 | 117.2 ± 19.9 |
| DBP (mm Hg) | 69.7 ± 10.9 | 68.3 ± 8.4 | 66.2 ± 21.9 | 59.3 ± 19.8 |
| MBP (mm Hg) | 79.7 ± 39.6 | 76.3 ± 38.1 | 92.8 ± 21.3 | 79.8 ± 23.8 |
| BR (rep/min) | 13.8 ± 7.9 | 17.8 ± 6.1 | 15.8 ± 6.9 | 16.3 ± 6.3 |
| ETCO₂ | 32.5 ± 2.7 | 32.7 ± 5.6 | 33.8 ± 3.2 | 32.5 ± 3.4 |
| SpO₂ (%) | 96.8 ± 0.6 | 98.4 ± 0.7 | 97.2 ± 0.6 | 98.2 ± 0.6 |

No statistically significant differences are observed from the parameters monitored after the administration of the inductor in none of the two groups analyzed. No signs of discomfort or pain were either observed during the administration of the inductor in the ASA II group.

No effect adverse or sequels were observed in any of the animals treated and no signs of phlebitis were registered in the subsequent controls.

### Conclusions:

Results shows that the use of the induction solution according to this instant invention is able to induce anesthesia in pre-medicated dogs and make the oro-tracheal intubation without complications at the dose employed. We were also able to discard the occurrence of adverse events with the use of the formulation of this invention.

## Claims

1. **PROPOFOL TRANSPARENT ANESTHETIC SOLUTION, WITH LOW VENOUS IRRITATION THRESHOLD characterized by** being composed, at least, by 90% to 55% of Propofol, dissolved in 10% to 45% of Macrogol 15-hydroxystearate (Solutol).

2. **PROPOFOL TRANSPARENT ANESTHETIC SOLUTION, WITH LOW VENOUS IRRITATION THRESHOLD** in accordance with what has been claimed in 1, **characterized by** having 25 % of Macrogol 15-hydroxystearate (Solutol) for each 10.815 mg/ ml of Propofol.

3. **PROPOFOL TRANSPARENT ANESTHETIC SOLUTION, WITH LOW VENOUS IRRITATION THRESHOLD** in accordance what has been claimed in 1 and 2, **characterized by** having 0.0515 mg/ml of EDTA for each 10.815 mg/ ml of Propofol.

## Patentansprüche

1. Transparente Propofol Narkoselösung mit geringer Venenreizung, **dadurch gekennzeichnet, dass** die aus wenigstens 90% - 55% Propofol besteht, gelöst in 10% - 45% Macrogol 15-Hydroxystearat (Solutol).

2. Transparente Propofol Narkoselösung mit geringer Venenreizung gemäß Anspruch 1, **dadurch gekennzeichnet, dass** sie 25% Macrogol 15-Hydroxystearat (Solutol) auf jeweils 10,815 mg/ml Propofol hat.

3. Transparente Propofol Narkoselösung mit geringer Venenreizung gemäß Anspruch 1 und 2, **dadurch gekennzeichnet, dass** sie 0,0515 mg/ml EDTA auf jeweils 10,815 mg/ml Propofol hat.

## Revendications

1. Solution anesthésique transparente de Propofol, avec un seuil de faible irritation veineuse, **caractérisée en ce qu'**elle est composée, au moins, par 90 % à 55 % de Propofol, dissous dans 10 % à 45 % de Macrogol 15-hydroxystéarate (Solutol).

2. Solution anesthésique transparente de Propofol, avec un seuil de faible irritation veineuse conformément à ce qui a été revendiqué dans 1, **caractérisée en ce qu'**elle a 25 % de Macrogol 15-hydroxystéarate (Solutol) pour chaque 10, 815 mg/ml de Propofol.

3. Solution anesthésique transparente de Propofol, avec un seuil de faible irritation veineuse conformément à ce qui a été revendiqué dans 1 et 2, **caractérisée en ce qu'**elle a 0,0515 mg/ml de EDTA pour chaque 10,815 mg/ml de Propofol.
